# EUROPÄISCHE PATENTSCHRIFT

(11) **EP 3 121 125 B1**
(45) Veröffentlichungstag und Bekanntmachung des Hinweises auf die Patenterteilung: **23.01.2019**
(21) Anmeldenummer: 16183574.9
(22) Anmeldetag: 11.07.2012
(51) Int. Cl.: B65B 55/10, A61L 2/08, A61L 2/10, A61L 2/18, A61L 2/22, B65B 55/04, B65B 55/08, B65D 25/00, B65D 59/04

(54) **PACKUNGSMANTEL ZUR HERSTELLUNG EINER VERPACKUNG FÜR NAHRUNGSMITTEL**
PACKAGING SHEATH FOR FOOD PACKAGE PRODUCTION
ENVELOPPE D'EMBALLAGE DESTINÉE À FABRIQUER UN EMBALLAGE POUR PRODUITS ALIMENTAIRES

(30) Priorität: 31.08.2011 DE 102011111523
(43) Veröffentlichungstag der Anmeldung: 25.01.2017
(62) Teilanmeldung aus: 12738076.4
(73) Patentinhaber: SIG Technology AG, 8212 Neuhausen am Rheinfall (CH)
(72) Erfinder: Flörke, Rudolf, 52428 Jülich (DE); Geissler, Hanno, 47802 Krefeld (DE); Mainz, Hans-Willi, 52525 Heinsberg (DE)
(74) Vertreter: Cohausz & Florack

(56) Entgegenhaltungen:
- EP-A1- 0 394 734
- US-A- 3 923 238
- US-A- 4 631 173

## Beschreibung

Die Erfindung betrifft eine Gruppe von Packungsmänteln zur Herstellung von Verpackungen für Nahrungsmittel, insbesondere flüssige Lebensmittel.

Bei der Herstellung von Mehrschichtverbundpackungen, beispielsweise Getränkepackungen, werden unterschiedliche Verfahren angewandt. Beispielsweise erfolgt die Herstellung von Packungen aus einzelnen Zuschnitten aus Papier/Kunststoff-Verbundmaterial. Hier werden zunächst aus einer Rolle an Verbundmaterial einzelne Zuschnitte gewonnen und diese anschließend mit einer Längsnaht versehen, so dass ein sog. Packungsmantel (engl.: ,sleeve') entsteht. Die Längsnähte werden durch Faltung und Versiegeln des Verbundmaterials derart erzeugt, dass ein in die Verpackung zu füllendes Produkt nicht mit einer offenen Kante des Verbundmaterials in Kontakt treten kann. Ein solcher Kontakt könnte zu einer Aufweichung des Verpackungsmaterials und zu einer Kontamination des abgefüllten Lebensmittels führen.

Die weitere Verarbeitung derart hergestellter Packungsmäntel, also das einseitige Verschließen auf der Ober- oder Unterseite der späteren Verpackung, das Entkeimen, das Befüllen und erneute Verschließen erfolgt meist direkt in der Füllmaschine.

Bei der Herstellung der Packung wird der Packungsmantel zunächst an der Ober- oder Unterseite verschlossen. Anschließend wird das Packungsinnere gereinigt und gegebenenfalls desinfiziert, bevor der einseitig verschlossene Behälter der aseptischen Zone der Füllmaschine zugeführt wird. Dort erfolgt das Befüllen und Verschließen der Packung. Im Anschluss erfolgt die endgültige Formgebung der Packung. Ein solches Sterilisationsverfahren ist unter anderem in der DE 32 35 476 A1 beschrieben.

Unabhängig vom Herstellungsverfahren, erfolgt das Verschließen der Packung in der Regel durch Zusammenpressen und Versiegeln der Packstoffkanten beispielsweise mittels einer Sonotrode und einem Amboss. Es sind auch andere Verfahren zum Verschließen der Packung bekannt, beispielsweise elektromagnetische Induktion oder Heißluft in Verbindung mit mechanischem Verpressen.

Das Verschließen eines einseitig offenen, gefüllten Packungsmantels birgt das Risiko, dass insbesondere beim Versiegeln mit Ultraschall, aus den offenen Schnittkanten Staub aus dem Verpackungsmaterial geschleudert werden kann und dieser sowohl den aseptischen Bereich als auch die offenen Verpackungen verunreinigen kann.

Aus dem Stand der Technik sind verschiedene Verfahren zum Versiegeln der Kanten von Verpackungsrohlingen aus Karton bekannt. Das Versiegeln hat zum Ziel, das Eindringen von Flüssigkeit nach Fertigstellung der Verpackung in innen liegende Kanten der Verpackung zu verhindern.

Die DE 30 11 630 A1 offenbart ein Verfahren zur Behandlung von Verpackungsstirnseiten mit einem Öl, das in die Stirnseiten eindringt. Dabei dringt das Öl möglichst in alle Poren ein und kann durch Hitzeeinwirkung teilweise polymerisiert werden. Dadurch nimmt es an Viskosität zu.

Die WO 96/18544 A1 beschreibt eine Methode zum Versiegeln von Kanten von Verpackungen mit Wachs, Kunststoff, Klebstoff oder Schmelzkleber. Die Versiegelung wird dabei entweder durch eine Düse, durch Eintauchen oder Aufsprühen aufgetragen. Anschließend kann die Versiegelung durch Hitze ausgehärtet werden.

Aus der US 3 187 480 A ist es bekannt, einen Stapel von Kartonrohlingen zusammenzupressen und zum Schutz der Schnittkanten in ein heißes Bad aus Imprägnierlösung einzutauchen. Als Imprägnierlösung sind Glycin und Rizinusöl offenbart.

Es ist auch bereits aus der US 4 631 173 A bekannt, oben offene Behälter vor ihrer Befüllung mit einem Produkt mittels einem Sterilisationsmittel, welches als heißes Dampf-Gasgemisch in das Packungsinnere eingebracht wird, zu sterilisieren. Dabei wird das Dampf-Gasgemisch über die Packungsränder umgelenkt und kommt dabei nur sehr kurz mit den oben offenen Kanten des Behälters in Berührung. Das bekannte Verfahren dient daher zur Sterilisation der oben offenen Kanten und zur Sterilisation des Behälterinneren.

Davon ausgehend liegt der Erfindung die Aufgabe zugrunde, eine eingangs genannte und zuvor näher beschriebene Gruppe von Packungsmänteln derart mit einem Entkeimungsmittel zu behandeln, dass das Risiko der Kontamination der Verpackungen, des Produktes und des aseptischen Bereichs einer Abfüllanlage beim Befüllen und Verschließen der Verpackungen durch die offenen Schnittkanten der Packungsmäntel reduziert wird.

Diese Aufgabe wird durch Anspruch 1 gelöst.

Weil die Packungsmäntel bei der Behandlung mit den oberen und/oder unteren Schnittkanten nach oben gerichtet sind, dringt das Entkeimungsmittel besonders gut in die oberste Kartonschicht des Verpackungsmaterials ein. Bevorzugt erfolgt das Eindringen des Entkeimungsmittels in einem Bereich von 1 mm bis 2 mm in das Verpackungsmaterial (von der Schnittkante aus gemessen).

Die Eindringtiefe des Entkeimungsmittels in die Kanten des Verpackungsmaterials beträgt mindestens 1 mm, bevorzugt zwischen 1 und 2 mm. Die Kanten können, abhängig vom Behandlungsmittel, durch dieses ausgeblichen werden, was insbesondere dann der Fall ist, wenn das Verpackungsmaterial aus ungebleichtem Karton besteht oder diesen enthält. Durch das Behandeln der offenen Schnittkanten mit Entkeimungsmittel und das Eindringen des Entkeimungsmittels in das Verpackungsmaterial werden die offenen Kanten und auf diesen abgelagerte und/oder von diesen aufgenommene Partikel, insbesondere Staub, wirkungsvoll entkeimt und damit das Kontaminationsrisiko reduziert.

Erfindungsgemäß sind während des Aufbringens eine Mehrzahl von Packungsmänteln flach gefaltet und aufrecht stehend zu einer Gruppe zusammengefasst. Ziel dieses Vorgehens ist ein möglichst effizienter Einsatz des Entkeimungsmittels.

Das Verpackungsmaterial für die Packungsmäntel zur Herstellung von Verpackungen für Nahrungsmittel, insbesondere flüssige Lebensmittel ist bevorzugt Verbundverpackungsmaterial, insbesondere Papier/Kunststoff-Verbundverpackungsmaterial.

Bevorzugt wird die Gruppe von Packungsmänteln nach der Behandlung in einer Umverpackung verpackt. Nach dem Transport zur Füllmaschine wird der Packungsmantel aufgefaltet, geformt und auf der Ober- oder Unterseite verschlossen. Die Behandlung kann auch nach dem einseitigen Verschließen des Packungsmantels angewendet werden. Die Sterilisation der Innenseite des Packungsmantels erfolgt, bevor er im aseptischen Bereich befüllt und verschlossen wird.

Das verwendete Entkeimungsmittel ist bevorzugt flüssig. Das Entkeimungsmittel hat bei der Behandlung bevorzugt Raumtemperatur. Bevorzugt enthält das Entkeimungsmittel Wasserstoffperoxid oder Peressigsäure. Der Wasserstoffperoxid-Gehalt beträgt 35% oder der Wasserstoffperoxid-Gehalt beträgt nur 2% und das Entkeimungsmittel wird anschließend mit einer UV-Strahlung bestrahlt.

Zusätzlich oder alternativ ist das Entkeimungssmittel ein Gemisch, das Wasserstoffperoxid und Alkohol enthält und/oder zusätzlich eine Imprägnierlösung und/oder eine Hydrophobierungslösung. Weiterhin kann das Entkeimungssmittel einen Indikator, beispielsweise Farbpigmente, enthalten. Ferner ist bevorzugt, dass das erfindungsgemäße Verfahren eine Reduzierung der Gesamtkoloniezahl bestimmt nach DIN 54379 von mindestens log 2 (∼99%) erreicht.

Vorteilhaft ist es ebenfalls, wenn das Entkeimungsmittel ein Benetzungsmittel, beispielsweise eine oberflächenaktive Substanz, zum verbesserten Eindringen des Entkeimungsmittels in das Verpackungsmaterial enthält. Ein bevorzugtes Mischverhältnis ist: Behandlungsmittel 1000 : Benetzungsmittel 1.

Das Entkeimungssmittel wird bevorzugt durch eine Düse auf die Schnittkanten der Packungsmäntel aufgebracht. Das Aufbringen des Entkeimungsmittels auf die offenen Schnittkanten kann jedoch auch durch Tauchen der Schnittkanten in ein Bad, über Bürsten, Rollen und/oder Walzen erfolgen.

Die während der Behandlung entstehenden Dämpfe werden abgesaugt, insbesondere von einer Absaugung aufgenommen und einer Abluftreinigung und/oder einem Abluftwäscher zugeführt. Da von den Dämpfen ein Gesundheitsrisiko ausgehen kann, kann durch eine Absaugung die Belastung für die Umwelt reduziert werden. Hier sind insbesondere die einschlägigen Vorschriften zu beachten.

Die oberen und/oder unteren offenen Schnittkanten werden mit dem Entkeimungsmittel imprägniert. Dem Fachmann sind aus dem Stand der Technik verschiedene Imprägniermittel für das Imprägnieren von Schnittkanten bekannt.

Aus der DE 25 22 546 A1 ist für sich ein Verfahren zur Behandlung der Stirnseiten einer Rolle bzw. eines Zuschnitts mit Imprägnierlösung bekannt, um das Eindringen von Flüssigkeit in die offene Kante zu verhindern.

Unmittelbar nach der Behandlung wird die Umverpackung, welche eine bestimmte Anzahl von Packungsmänteln enthält, verschlossen. Dies verhindert, dass sich Fremdkörper, insbesondere Staub, neu an den Kanten anlagern und diese nicht wirksam entkeimt werden und beim Verschließen der Packungsmäntel nach dem Befüllen in der Füllmaschine ein Kontaminationsrisiko darstellen. Hierbei können auch die Innenseiten der oberen und/oder unteren Flächen der Umverpackung mit dem Entkeimungsmittel behandelt werden. Dies hat den Vorteil, dass nach dem Verschließen eine Re-Kontamination bereits behandelter Flächen reduziert wird. Diese Behandlung kann beispielsweise in der gleichen Vorrichtung erfolgen.

Ein Behandeln von Packungsmänteln im Endbereich der oberen und/oder unteren Schnittkanten mit Entkeimungsmittel entkeimt diese und dort abgelagerte Partikel wirkungsvoll, so dass beim Schließen der Packungsmäntel nach dem Befüllen von diesen und auf diesen abgelagertem Staub kein Keimrisiko für den aseptischen Bereich und nachfolgende Packungsmäntel mehr ausgeht.

Die Erfindung wird nachfolgend anhand einer lediglich bevorzugte Ausführungsbeispiele darstellenden Zeichnung näher erläutert.

In der Zeichnung zeigen
- Fig. 1: eine Vorrichtung zum Aufbringen eines Entkeimungssmittels auf offene Schnittkanten eines Mantels aus Verpackungsmaterial mit zwei Behandlungsstationen und
- Fig. 2: eine Vorrichtung zum Aufbringen eines Entkeimungssmittels auf offene Schnittkanten eines Mantels aus Verpackungsmaterial mit vier Behandlungsstationen.

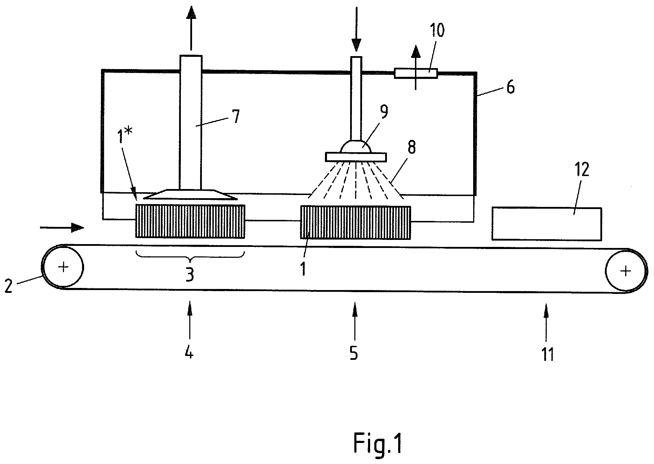

Fig. 1 zeigt eine Vorrichtung zur Behandlung der oberen Schnittkanten 1* von Packungsmänteln 1. Die Packungsmäntel 1 befinden sich als Gruppe aus mehreren Packungsmänteln 1 zusammengefasst in einer offenen Umverpackung 3. Es ist ein Förderband 2 gezeigt, auf dem die offene Umverpackung 3 zwei Behandlungsstationen 4 und 5 durchläuft. Beide Behandlungsstationen sind durch eine gemeinsame Einhausung 6 von der Umgebung getrennt.

In der ersten (optionalen) Behandlungsstation 4 werden Partikel, insbesondere Staub, mit Hilfe einer Staubabsaugung 7 mindestens von den oberen Kanten der Packungsmäntel 1 abgesaugt. In der nächsten Behandlungsstation 5 wird ein ein Entkeimungsmittel 8 durch eine Düse 9 mindestens auf die Kanten der Packungsmäntel 1 aufgetragen und kann in diese eindringen.

Das Entkeimungsmittel 8 kann zusätzlich ein Benetzungsmittel enthalten. Über der Behandlungsstation 5 kann eine weitere Absaugung 10 zum Absaugen von bei der Behandlung auftretenden Dämpfen vorgesehen sein. Danach verlassen die Packungsmäntel 1 in der offenen Umverpackung die Einhausung 6 wonach in einer Packstation 11 die Umverpackung verschlossen wird. In der verschlossenen Umverpackung 12 erfolgt der Weitertransport der Packungsmäntel 1.

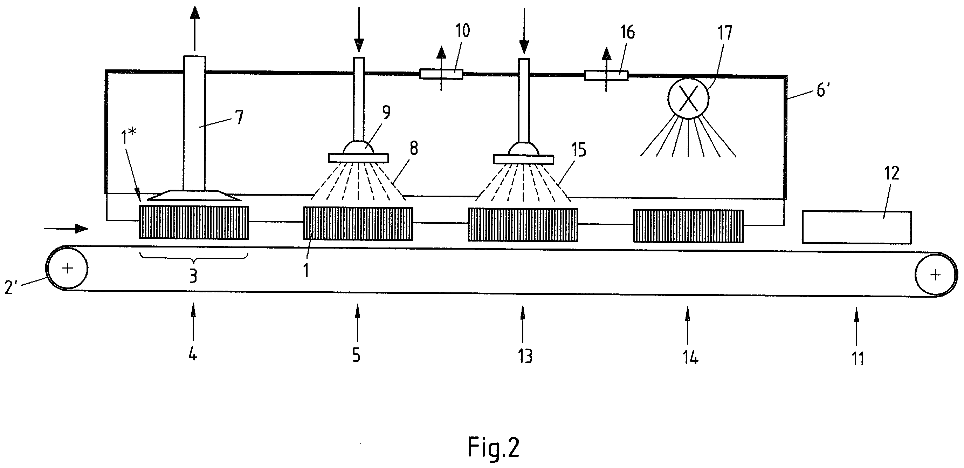

Fig. 2 zeigt ein weiteres Ausführungsbeispiel zur Behandlung von Packungsmänteln 10. An die beiden bereits in Fig. 1 gezeigten Behandlungsstationen 4 und 5 schließen sich eine dritte Behandlungsstation 13 und eine vierte Behandlungsstation 14 an, bevor die offene Umverpackung 3 in einer Packstation 11 verschlossen wird. In diesem Ausführungsbeispiel sind alle vier Behandlungsstationen 4, 5, 13, 14 über ein Förderband 2' durch eine Einhausung 6' von der Umgebung getrennt.

Die dritte Behandlungsstation 13 ist optional und imprägniert mindestens die Kanten 1* der Packungsmäntel 1 mit einem Imprägniermittel 15. Über dieser Behandlungsstation 13 kann ebenfalls eine Absaugung 16 zum Absaugen von bei der Behandlung auftretenden Dämpfen vorgesehen sein. Diese kann mit der Absaugung 10 von der zweiten Behandlungsstation 5 verbunden sein. Die Absaugungen 10, 16 können mit einer nicht gezeigten Abluftreinigungseinrichtung oder einem nicht gezeigten Abluftwäscher verbunden sein.

In der vierten Behandlungsstation 14 werden mindestens die offenen oberen Kanten der Packungsmäntel 1 von einer Strahlenquelle 17 mit UV- und/oder Beta- und/oder Gamma-Strahlen bestrahlt. Danach verlassen die behandelten Packungsmäntel 1 in der offenen Umverpackung 3 die Einhausung 6', werden verschlossen und die geschlossenen Umverpackungen 12 können zum Abfüllort transportiert werden.

## Patentansprüche

1. Gruppe von Packungsmänteln zur Herstellung von Verpackungen für Nahrungsmittel, insbesondere flüssige Lebensmittel,
**dadurch gekennzeichnet, dass**
jeder Packungsmantel (1) flach gefaltet ist, dass die Gruppe eine Mehrzahl von flach gefalteten und aufrecht zusammengefasst stehenden Packungsmänteln (1) aufweist, dass die oberen und/oder unteren Schnittkanten (1*) nach oben gerichtet sind und dass die Schnittkanten (1*) mittels eines Entkeimungsmittels (8) behandelt sind, so dass das Entkeimungsmittel mindestens in den Endbereich der oberen und/oder unteren Schnittkanten (1*) eingedrungen ist.

2. Gruppe von Packungsmänteln nach Anspruch 1,
**dadurch gekennzeichnet, dass**
jeder Packungsmantel (1) aus Verpackungsmaterial, insbesondere Papier/Kunststoff-Verbundmaterial besteht.

3. Gruppe von Packungsmänteln nach Anspruch 1 oder 2,
**dadurch gekennzeichnet, dass** die Packungsmäntel (1) der Gruppe in einer Umverpackung (12) verpackt sind und dass die Umverpackung (12) mit den Packungsmänteln (1) verschlossen ist.

4. Gruppe von Packungsmänteln nach einem der Ansprüche 1 bis 3,
**dadurch gekennzeichnet, dass**
das verwendete Entkeimungsmittel (8) Wasserstoffperoxid oder Peressigsäure enthält.

5. Gruppe von Packungsmänteln nach einem der Ansprüche 1 bis 4,
**dadurch gekennzeichnet, dass**
das verwendete Entkeimungsmittel (8) zusätzlich ein Benetzungsmittel enthält.

## Claims

1. Group of carton sleeves for producing packaging for food products, in particular liquid food,
**characterised in that**
each carton sleeve (1) is folded flat, **in that** the group has a plurality of carton sleeves (1) which are folded flat and combined so as to stand upright, **in that** the upper and/or lower cut edges (1*) are directed upwards and **in that** the cut edges (1*) are treated by means of a sterilising agent (8) so the sterilising agent has penetrated at least into the end region of the upper and/or lower cut edges (1*).

2. Group of carton sleeves according to Claim 1,
**characterised in that**
each carton sleeve (1) is composed of packaging material, in particular paper/plastic composite material.

3. Group of carton sleeves according to Claim 1 or 2,
**characterised in that**
a plurality of carton sleeves (1) of the group are packed in an outer packaging (12) before or after treatment by the disinfectant agent (8), and **in that** the outer packaging (12) with the carton sleeves (1) is closed after treatment with the disinfectant agent (8).

4. Group of carton sleeves according to any one of Claims 1 to 3,
**characterised in that**
the sterilising agent (8) used contains hydrogen peroxide or peracetic acid.

5. Group of carton sleeves according to any one of Claims 1 to 4,
**characterised in that**
the sterilising agent (8) used also contains a wetting agent.

## Revendications

1. Groupe d'enveloppes d'emballage destiné à la préparation d'emballages pour produits alimentaires, en particulier de la nourriture liquide, **caractérisé en ce que** chaque enveloppe d'emballage (1) est plissée à plat, **en ce que** le groupe comprend plusieurs enveloppes d'emballage (1) plissées à plat et assemblées verticalement, **en ce que** les bords de coupe (1*) supérieurs et/ou inférieurs sont dirigés vers le bas et **en ce que** les bords de coupe (1*) sont traités par un agent désinfectant (8), de sorte que l'agent désinfectant ait pénétré au moins dans la zone d'extrémité des bords de coupe (1*) supérieurs et/ou inférieurs.

2. Groupe d'enveloppes d'emballage selon la revendication 1, **caractérisé en ce que** chaque enveloppe d'emballage (1) consiste en un matériau d'emballage, en particulier un matériau composite papier/matière plastique.

3. Groupe d'enveloppes d'emballage selon la revendication 1 ou 2, **caractérisé en ce que** les enveloppes d'emballage (1) du groupe sont emballées dans un suremballage (12) et **en ce que** le suremballage (12) est fermé avec les enveloppes d'emballage (1).

4. Groupe d'enveloppes d'emballage selon l'une des revendications 1 à 3, **caractérisé en ce que** l'agent désinfectant (8) utilisé contient le peroxyde d'hydrogène ou l'acide peracétique.

5. Groupe d'enveloppes d'emballage selon l'une des revendications 1 à 4, **caractérisé en ce que** l'agent désinfectant (8) utilisé contient en outre, un agent mouillant.
